(19) Europäisches Patentamt
European Patent Office
Office européen des brevets

(11) Veröffentlichungsnummer : **0 013 360**
**B1**

(12) # EUROPÄISCHE PATENTSCHRIFT

(45) Veröffentlichungstag der Patentschrift : **07.04.82**

(21) Anmeldenummer : **79105052.9**

(22) Anmeldetag : **10.12.79**

(51) Int. Cl.³ : **C 07 D249/08**, C 07 D233/56, C 07 D231/12, A 01 N 43/48, A 01 N 43/64

(54) **N-substituierte Carbonsäureanilide, Verfahren zu ihrer Herstellung sowie ihre Verwendung als Fungizide.**

(30) Priorität : **18.12.78 DE 2854598**

(43) Veröffentlichungstag der Anmeldung : **23.07.80 (Patentblatt 80/15)**

(45) Bekanntmachung des Hinweises auf die Patenterteilung : **07.04.82 Patentblatt 82/14**

(84) Benannte Vertragsstaaten : **AT BE CH DE FR GB IT LU NL SE**

(56) Entgegenhaltungen :
**DE - A - 2 648 008**
**DE - A - 2 704 281**
**EP - A - 0 006 540**
**EP - A - 0 007 080**
**EP - A - 0 008 091**
**K.H. Bückel, Pflanzenschutz- und Schädlingsbekämpfung (Thieme-Verlag, Stuttgart, 1977), S. 150-151**

(73) Patentinhaber : **BASF Aktiengesellschaft**
**Carl-Bosch-Strasse 38**
**D-6700 Ludwigshafen (DE)**

(72) Erfinder : **Plath, Peter, Dr. Dipl.-Chem.**
**Berner Weg 24**
**D-6700 Ludwigshafen (DE)**
Erfinder : **Eicken, Karl, Dr. Dipl.-Chem.**
**Waldstrasse 63**
**D-6706 Wachenheim (DE)**
Erfinder : **Rohr, Wolfgang, Dr. Dipl.-Chem.**
**Gontardstrasse 4**
**D-6800 Mannheim 1 (DE)**
Erfinder : **Zeeh, Bernd, Dr. Dipl.-Chem.**
**Thorwaldsenstrasse 5**
**D-6700 Ludwigshafen (DE)**
Erfinder : **Pommer, Ernst-Heinrich, Dr.**
**Berliner Platz 7**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 013 360 B1

N-substituierte Carbonsäureanilide, Verfahren zu ihrer Herstellung sowie ihre Verwendung als Fungizide

Die vorliegende Erfindung betrifft neue wertvolle N-substituierte Carbonsäureanilide, Verfahren zu ihrer Herstellung sowie ihre Verwendung als Fungizide.

Es ist bekannt, N-Trichlormethylthio-phthalimid als Fungizid zu verwenden (Chemical Week, 1972, June 21, Seite 63). Außerdem sind N-Azolylmethyl-chloracetanilide als Herbizide bekannt geworden (DE-A 26 48 008 und DE-A 27 04 281). Es findet sich kein Hinweis auf eine fungizide Wirkung.

Es ist ferner bekannt, Imidazol- oder Triazolderivate als Fungizide zu verwenden (K.H. Büchel, Pflanzenschutz und Schädlingsbekämpfung Thieme-Verlag, Stuttgart 1977 Seiten 150-151). Die dort beschriebenen Verbindungen enthalten jedoch keinen substituierten Säureanilidrest und sind daher in ihrer chemischen Struktur von den neuen Verbindungen völlig verschieden.

Es wurde nun gefunden, daß neue N-substituierte Carbonsäureanilide der Formel I

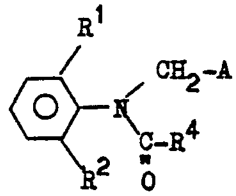

(I)

worin

$R^1$ $C_1$-$C_4$-Alkyl,

$R^2$ $C_1$-$C_4$-Alkyl

$R^4$ Methoxy, Ethoxy, Isopropoxy, Phenoxy, Benzyl oder 1-Phenyläthyl oder ggf. durch Fluor, Chlor, Brom, Jod, Methyl, Methoxy oder Nitro einfach substituiertes Phenyl bedeutet oder

$R^4$ den Rest $-CH_2-Y$ bedeutet, wobei Y Methoxy, Ethoxy, Phenoxy oder Benzyloxy bedeutet und

A ein über ein Stickstoffatom gebundenes Pyrazol, Imidazol oder 1,2,4-Triazol bedeutet und deren Säureadditionsprodukte eine gute fungizide Wirkung haben.

Die Herstellung der neuen Verbindungen kann in der Weise erfolgen, daß man ein N-Halogenmethylcarbonsäureanilid der Formel II

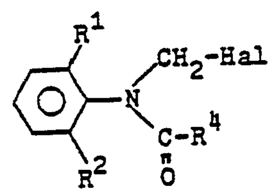

(II)

in welcher $R^1$, $R^2$ und $R^4$ und n die oben angegebene Bedeutung haben, und Hal Halogen, insbesondere Chlor oder Brom bedeutet, mit Azolen der Formel III

$$A—M \qquad \text{(III)}$$

in welcher

A die oben angegebene Bedeutung hat und

M Wasserstoff oder ein Alkalimetall bedeutet,

gegebenenfalls in Gegenwart eines Verdünnungsmittels und gegebenenfalls in Gegenwart eines Säurebindemittels umsetzt und gegebenenfalls an die so erhaltene Verbindung anschließend eine Säure oder ein Metallsalz addiert.

Im Falle der Umsetzung von 2,6-Dimethyl-N-chlormethyl-benzanilid und Imidazol kann der Reaktionsablauf durch das folgende Reaktionsschema wiedergegeben werden

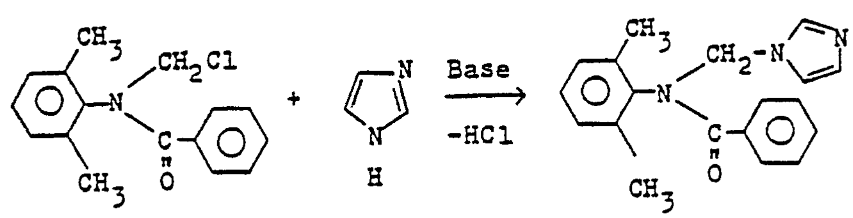


In der Formel I bedeutet $R^1$ vorzugsweise Methyl oder Äthyl, während $R^2$ bevorzugt Methyl, Äthyl,

Isopropyl oder tert.-Butyl bedeutet.

Hal steht in Formel II bevorzugt für Chlor, kann jedoch auch für Brom stehen.

$R^4$ in Formel I steht beispielsweise für Benzyl, 1-Phenyläthyl

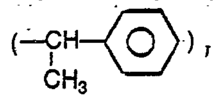

Phenyl, 2-Methylphenyl, 3-Methylphenyl, 4-Methylphenyl, 2-Chlorphenyl, 3-Chlorphenyl, 4-Chlorphenyl, 2-Fluorphenyl, 3-Fluorphenyl, 4-Fluorphenyl, 3-Bromphenyl, 2-Nitrophenyl, 3-Nitrophenyl, 4-Nitrophenyl, 2-Methoxyphenyl, 3-Methoxyphenyl, 4-Methoxyphenyl, 2-Jodphenyl.

$R^4$ steht außerdem für den Rest $-CH_2-Y$, wobei Y den Rest Methoxy, Äthoxy, Benzyloxy, Phenoxy bedeutet.

$R^4$ steht außerdem für den Rest $-O-R^5$, wobei $R^5$ für Methyl, Äthyl, Isopropyl oder Phenyl steht.

Die Herstellung der N-Halogenmethylcarbonsäureanilide der Formel II erfolgt durch Addition eines Säurehalogenids $R^4-CO-Hal$ an eine Schiff'sche Base der Formel VI

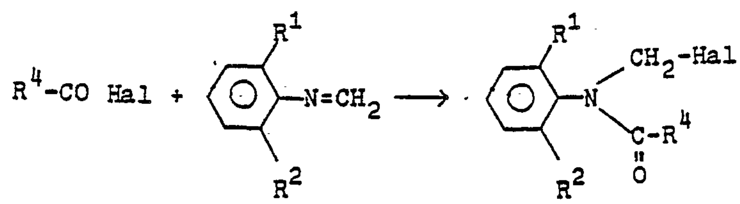

(VI) (II)

wobei Hal Halogen bedeutet und $R^1$, $R^2$ und $R^4$ die oben genannten Bedeutungen haben.

Diese Verfahrensvariante ist Gegenstand der US-Patentschriften 3 630 716 und 3 637 847 und der DE-AS-15 42 950, sofern $R^4-CO$ Hal ein Säurehalogenid einer Benzoesäure oder einer Alkancarbonsäure ist.

Die US-Patentschriften 3 714 299 und 3 810 981 beschreiben ferner Verbindungen der Formel II, in denen $R^4$ ein Alkoxy-, ein Alkoxyalkyl-, Alkenyl- oder Cycloalkylrest ist.

Ferner wird in der deutschen Offenlegungsschrift 21 19 518 die Herstellung von N-Aryl-N-chlormethylcarbamaten durch Chlormethylierung von N-Arylcarbamaten beschrieben, wobei ausdrücklich betont wird, daß nach dem dort beschriebenen Verfahren auch Verbindungen der Formel IIa (s.u.) erhältlich sind, die nicht durch Addition von Chlorkohlensäure-O-arylestern an Hexahydrotriazine (= Trimere Schiff'sche Basen) zugänglich seien. Wie in den folgenden Beispielen gezeigt wird, gelingt jedoch die Addition von Chlorkohlensäure-estern auch dann gut an 2,6-Dialkyl-N-methylenaniline VI, wenn es sich um Chlorkohlensäure-O-arylester handelt. Die Umsetzung erfolgt gemäß folgendem Schema.

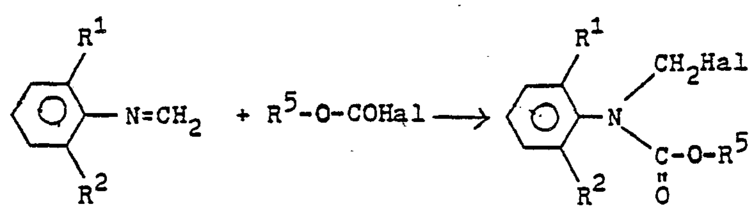

(VI) (IIA)

Obwohl die Umsetzung von z.B. Benzoylchlorid mit 2,6-Dimethyl-N-methylenanilin in den zitierten US-Patentschriften beansprucht wird, ist in der Literatur keine Angabe über Eigenschaften von und Umsetzungen mit N-Chlormethyl-2,6-dimethyl-N-benzoyl-anilin zu finden. Eines der folgenden Beispiele zeigt diese Umsetzung. Die als Ausgangsprodukte benötigten N-Chlormethyl-carbonsäureanilide sind in der folgenden Tabelle I aufgeführt.

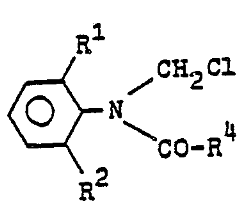

Tabelle I

| Lfd.Nr. | $R^1$ | $R^2$ | $R^4$ | Physikalische Konstanten |
|---|---|---|---|---|
| 1 | $CH_3$ | $CH_3$ | Benzyl | 62-64 |
| 2 | $CH_3$ | $C_2H_5$ | Benzyl | |
| 3 | $C_2H_5$ | $C_2H_5$ | Benzyl | |
| 4 | $CH_3$ | $CH_3$ | $-CH_2OCH_3$ | 70°C |
| 5 | $CH_3$ | $C_2H_5$ | $-CH_2OCH_3$ | Öl |
| 6 | $C_2H_5$ | $C_2H_5$ | $-CH_2OCH_3$ | |
| 7 | $CH_3$ | $CH_3$ | $CH_2-O-C_2H_5$ | |
| 8 | $CH_3$ | $CH_3$ | $-CH_2-O-CH_2-$ 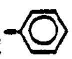 | |
| 9 | $CH_3$ | $CH_3$ | $CH_2-O-$ 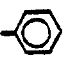 | Fp. 87°C |
| 10 | $C_2H_5$ | $C_2H_5$ | $CH_2-O-$ 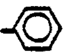 | |
| 11 | $CH_3$ | $CH_3$ | α-Phenyläthyl | |
| 12. | $C_2H_5$ | $C_2H_5$ | α-Phenyläthyl | $n_D^{23}$ = 1,5547 |
| 13 | $CH_3$ | $CH_3$ | Phenyl | Fp. 133°C |
| 14 | $CH_3$ | $C_2H_5$ | Phenyl | |
| 15 | $C_2H_5$ | $C_2H_5$ | Phenyl | Fp. 98-99°C |
| 16 | $C_3H_7$-i | $C_3H_7$-i | Phenyl | Fp. 107-108°C |
| 17 | $CH_3$ | $CH_3$ | 2-Methylphenyl | Fp. 118-119°C |
| 18 | $CH_3$ | $CH_3$ | 3-Methylphenyl | Fp. 133-135°C |
| 19 | $CH_3$ | $CH_3$ | 4-Methylphenyl | Fp. 119-120°C |
| 20 | $CH_3$ | $CH_3$ | 2-Chlorphenyl | Fp. 93°C |
| 21 | $CH_3$ | $CH_3$ | 3-Chlorphenyl | Fp. 145°C |
| 22 | $C_2H_5$ | $C_2H_5$ | 3-Chlorphenyl | |
| 23 | $CH_3$ | $CH_3$ | 4-Chlorphenyl | Fp. 126°C |
| 24 | $C_2H_5$ | $C_2H_5$ | 4-Chlorphenyl | |
| 25 | $CH_3$ | $CH_3$ | 2-Fluorphenyl | Fp. 86-88°C |
| 26 | $C_2H_5$ | $C_2H_5$ | 2-Fluorphenyl | |
| 27 | $CH_3$ | $CH_3$ | 3-Fluorphenyl | Fp. 114-115°C |
| 28 | $CH_3$ | $CH_3$ | 4-Fluorphenyl | Fp. 141-143°C |
| 29 | $CH_3$ | $CH_3$ | 3-Bromphenyl | |
| 30 | $CH_3$ | $CH_3$ | 2-Jodphenyl | |
| 31 | $CH_3$ | $CH_3$ | 2-Nitrophenyl | Fp. 205-207°C |
| 32 | $C_2H_5$ | $C_2H_5$ | 2-Nitrophenyl | |
| 33 | $CH_3$ | $CH_3$ | 3-Nitrophenyl | Fp. 152-154°C |

| Lfd.Nr. | $R^1$ | $R^2$ | $R^4$ | Physikalische Konstanten |
|---|---|---|---|---|
| 34 | $C_2H_5$ | $C_2H_5$ | 4-Nitrophenyl | Fp. 118-120°C |
| 35 | $CH_3$ | $CH_3$ | 2-Methoxyphenyl | |
| 36 | $CH_3$ | $CH_3$ | 3-Methoxyphenyl | Fp. 107-108°C |
| 37 | $CH_3$ | $CH_3$ | 4-Methoxyphenyl | Fp. 92-93°C |

Als Beispiele für die Ausgangsstoffe der Formel II seien weiterhin folgende N-Chlormethyl-N-Arylcarbamate der Formel VII genannt, die nach folgendem Schema hergestellt wurden

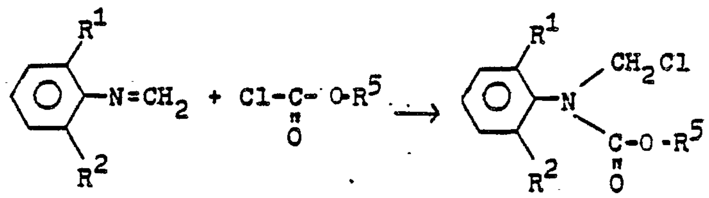

(VII)

wobei $R^1$, $R^2$ und $R^5$ die angegebene Bedeutung haben.

Tabelle II

| Lfd.Nr. | $R^1$ | $R^2$ | $R^5$ | Physikalische Konstante |
|---|---|---|---|---|
| 38 | $CH_3$ | $CH_3$ | $-CH_3$ | Öl |
| 39 | $C_2H_5$ | $C_2H_5$ | $CH_3$ | Öl |
| 40 | $CH_3$ | $CH_3$ | $C_2H_5$ | Öl |
| 41 | $C_2H_5$ | $C_2H_5$ | $C_2H_5$ | Öl |
| 42 | $CH_3$ | $CH_3$ | $C_2H_7$-i | |
| 43 | $C_2H_5$ | $C_2H_5$ | $CH_3H_7$-i | |
| 44 | $CH_3$ | $CH_3$ | Phenyl | Fp. 113-115°C |
| 45 | $C_2H_5$ | $C_2H_5$ | Phenyl | Fp. 94-95°C |
| 46 | $C_3H_7$-i | $C_3H_7$-i | Phenyl | Fp. 115-116°C |

Die außerdem als Ausgangsstoffe benötigten Schiff'schen Basen der Formel VI lassen sich nach bekannten Methoden (z.B. US 3 637 847) aus entsprechenden Anilinen und Paraformaldehyd in Toluol herstellen.

M steht in Formel III vorzugsweise für Wasserstoff, Natrium oder Kalium. Die Verbindungen der Formel III sind allgemein bekannte Verbindungen der organischen Chemie.

Für die Herstellungsverfahren kommen als Verdünnungsmittel vorzugsweise Toluol oder Essigsäureäthylester infrage. Es können jedoch auch andere (unter den Reaktionsbedingungen inerte) Lösungsmittel wie Diäthylketon, Propionitril, Acetonitril, Tetrahydrofuran, Dioxan, Xylol, Chloroform, Tetrachlorkohlenstoff, Chlorbenzol oder Dimethylformamid verwendet werden.

Als Säurebindemittel können anorganische Säureakzeptoren wie Alkalicarbonate, beispielsweise $Na_2CO_3$, $K_2CO_3$ oder $NaHCO_3$ oder tertiäre Amine, wie beispielsweise Triäthylamin, Dimethyl-benzylamin oder Pyridin verwendet werden. Es ist auch möglich, die entsprechenden Azole im Überschuß einzusetzen.

Die Reaktionstemperaturen können zwischen −10 und 150 °C variiert werden, bevorzugt wird der Bereich zwischen 20 bis 120 °C.

Bei der Herstellung der neuen Verbindungen setzt man auf 1 Mol der Verbindungen der Formel II vorzugsweise 1 bis 2 Mol des Azols der Formel III und 1 Mol Säurebinder ein, beziehungsweise auf 1 Mol

der Verbindungen gemäß Formel IV vorzugsweise 1 bis 1,2 Mol Carbonsäurehalogenid der Formel V und 1,1 bis 1,3 Mol Säurebinder.

Zur Isolierung der Verbindungen der Formel I wird das Reaktionsgemisch beispielsweise filtriert, das Filtrat mit Wasser gewaschen, getrocknet und eingeengt. Der Rückstand wird gegebenenfalls durch fraktionierte Kristallisation oder Destillation gereinigt.

Zur Herstellung von Säureadditionssalzen der Verbindungen der Formel I kommen bevorzugt physiologisch verträgliche Säuren infrage, beispielsweise Chlorwasserstoffsäure, ortho-Phosphorsäure, Salpetersäure, Maleinsäure, Zitronensäure, Essigsäure, p-Toluolsulfonsäure, Methansulfonsäure.

Zur Herstellung derartiger Salze bedient man sich der üblichen Methoden, beispielsweise Lösen der Verbindung nach Formel I in einem inerten Lösungsmittel wir Toluol, und anschließende Zugabe der Säure, Abfiltrieren, Isolieren und gegebenenfalls Reinigung durch Umkristallisieren.

Zur Herstellung von Metallsalz-Komplexverbindungen der Verbindungen der Formel I kommen vorzugsweise die Chloride oder Nitrate von Magnesium, Kupfer, Eisen, Mangan oder Nickel infrage. Man löst die Metallsalze in Äthanol und fügt anschließend die äquivalente Menge (oder weniger) der Verbindung der Formel I zu, isoliert das Komplexsalz durch Filtration und reinigt gegebenenfalls durch Umkristallisieren.

Beispiel 1

($C_{19}H_{20}N_4O_2$)

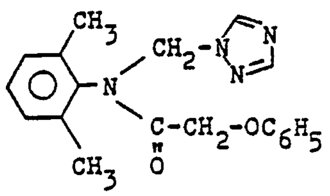

1. Zu einer Lösung von 34,1 g (0,2 Mol) Phenoxyacetylchlorid in 50 ml Ligroin tropft man langsam bei 0 bis 5 °C eine Lösung von 26,6 g (0,2 Mol) N-Methylen-2,6-dimethylanilin in 100 ml Toluol. Nach 16-stündigem Rühren bei Raumtemperatur wird vom ausgefallenen Produkt abgesaugt. Man isoliert nach Trocknen im Vakuum 47 g N-Chlormethyl-2,6-dimethyl-phenoxyacetanilid (78 % Ausbeute), Fp. 87 °C.

2. Man suspendiert 10,7 g (155 mMol) 1,2,4-Triazol in 100 ml Toluol und versetzt mit 23,5 g (77,5 mMol) N-Chlormethyl-2,6-dimethyl(phenoxyacetyl) anilid. Anschließend erhitzt man 1 Stunde auf 130 °C, so daß die Reaktionsmischung zum Sieden kommt. Nach dem Abkühlen wird filtriert, mit Toluol nachgewaschen und das Filtrat eingeengt. Der Filterrückstand wird verworfen. Man erhält nach dem Trocknen 17 g eines farblosen Feststoffs (65 % d.Th.) mit Schmelzpunkt 84 °C.

Beispiel 2

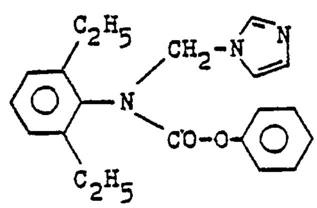

$C_{21}H_{23}N_3O_2$)

1. Zu einer Lösung von 31,3 g (0,2 Mol) Chlorameisensäurephenylester in 100 ml Ligroin gibt man bei 15 bis 20 °C unter leichter Kühlung eine Lösung von 32,2 g (0,2 Mol) N-Methylen-2,6-diäthylanilin in 50 ml Ligroin. Nach 16-stündigem Rühren isoliert man nach Absaugen und Trocknen 49,2 g (76 %) N-Chlormethyl-N-2,6-diäthylphenyl-carbaminsäure-O-phenylester vom Schmelzpunkt 94-95 °C.

2. 20 g (63 mMol) des obigen N-Chlormethylcarbamates werden in 100 ml Toluol mit 8,6 g (126 mMol) Imidazol gemischt; anschließend wird die Reaktionsmischung 1 Stunde zum Sieden erhitzt. Das Reaktionsgemisch wird anschließend noch heiß filtriert. Das nach dem Einengen des Lösungsmittels verbleidende Öl wird nach Anreiben mit Ligroin kristallin. Nach Absaugen und Trocknung erhält man 13,9 g (63 % d.Th.) N-Imidazolylmethyl-N-2,6-diäthylphenyl-carbaminsäure-O-phenyl-ester vom Schmelzpunkt 115 bis 116 °C.

Beispiel 3

Man vermischt 90 Gewichtsteile der nach Beispiel 1 erhaltenen Verbindung mit 10 Gewichtsteilen N-Methyl-α-pyrrolidon und erhält eine Mischung, die zur Anwendung in Form kleinster Tropfen geeignet ist.

Beispiel 4

20 Gewichtsteile der nach Beispiel 2 erhaltenen Verbindung werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 5

20 Gewichtsteile der nach Beispiel 2 erhaltenen Verbindung werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile der nach Beispiel 2 erhaltenen Verbindung werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 7

20 Gewichtsteile des nach Beispiel 2 erhaltenen Wirkstoffs werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 8

3 Gewichtsteile der nach Beispiel 1 erhaltenen Verbindung werden mit 97 Gewichtsteilen feinteiligem Kaolin innig vermischt. Man erhält auf diese Weise ein Stäubemittel, das 3 Gewichtsprozent des Wirkstoffs enthält.

Beispiel 9

30 Gewichtsteile der nach Beispiel 2 erhaltenen Verbindung werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 10

40 Gewichtsteile des nach Beispiel 1 erhaltenen Wirkstoffs werden mit 10 Teilen Natriumsalz eines Phenolsulfonsäure-Harnstoff-Formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 11

20 Teile des nach Beispiel 2 erhaltenen Wirkstoffs werden mit 2 Teilen Calciumsalz der Dodecylbenzolsulfonsäure, 8 Teilen Fettalkohol-polyglykoläther, 2 Teilen Natriumsalz eines Phenol-Harnstoff-Formaldehyd-Kondensats und 68 Teilen eines paraffinischen Mineralöls innig vermischt. Man erhält eine stabile ölige Dispersion.

Beispiel 12

a) Man löst 140,5 g (1 Mol) Benzoylchlorid in 200 ml Ligroin und tropft anschließend bei 5 bis 10 °C eine Lösung von 133 g (1 Mol) N-Methylen-2,6-dimethylanilin in Toluol zu. Nach 14 Stunden Rühren bei

25 °C wird der ausgefallene Feststoff durch Absaugen isoliert. Nach Trocknen im Vakuum erhält man 215 g (79 %) N-Chlormethyl-N-benzoyl-2,6-dimethylanilin mit Fp. 133 °C, Formel

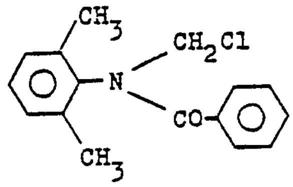

In analoger Weise wurde N-Chlormethyl-N-benzoyl-2,6-diäthylanilin vom Fp. 98 bis 99 °C erhalten.

b) Eine Lösung von 54,7 g (0,2 Mol) N-Chlormethyl-N-benzoyl-2,6-dimethylanilin in 250 ml Tetrahydrofuran wird bei 5 bis 10 °C zu einer Suspension von 13,8 g (0,2 Mol) Triazol in 100 ml Tetrahydrofuran und 20,2 g (0,2 Mol) Triäthylamin zugetropft. Nach 16 Stunden Rühren bei Raumtemperatur (25 °C) wird vom abgeschiedenen Triäthyl-ammoniumhydrochlorid abfiltriert. Nach Abziehen des Lösungsmittels wird der aus dem Filtrat isolierte Feststoff in 250 ml $CH_2Cl_2$ gelöst und mit 100 ml 5 %iger (Gew.%) Salzsäure ausgeschüttelt. Die organische Phase wird nach Trocknung über $Na_2SO_4$ eingeengt; der verbleibende Rückstand wird aus Toluol/Ligroin (3 : 2) umkristallisiert.

Man erhält 44,6 g (73 %) N-(Triazolylmethyl)-N-benzoyl-2,6-dimethylanilin der Formel

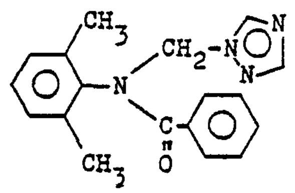

mit Schmelzpunkt 135 °C.

In gleicher Weise wurde N-(Triazolylmethyl)-N-benzoyl-2,6-diäthylanilin hergestellt, Fp. 112-115 °C.

In entsprechender Weise sind die in Tabelle III zusammengefaßten Produkte der Formel I erhalten worden, wobei die Substanzen aus Herstellungsbeispiel 1 und 2 nochmals mit aufgelistet wurden.

| Lfd.Nr. | $R^1$ | $R^2$ | $R^4$ | A | Physikalische Konstante (Fp. °C) |
|---|---|---|---|---|---|
| 47 | $CH_3$ | $CH_3$ | $CH_3$ | Pyrazol | 92 |
| 48 | $CH_3$ | $CH_3$ | $CH_3$ | 1,2,4-Triazol | 100 |
| 49 | $CH_3$ | $CH_3$ | $CH_3$ | Imidazol | |
| 50 | $CH_3$ | $C_2H_5$ | $CH_3$ | Imidazol | Öl |
| 51 | $CH_3$ | $C_2H_5$ | $CH_3$ | 1,2,4-Triazol | Öl |
| 52 | $C_2H_5$ | $C_2H_5$ | $CH_3$ | Pyrazol | 61 |
| 53 | $C_2H_5$ | $C_2H_5$ | $CH_3$ | Imidazol | Öl |
| 54 | $C_2H_5$ | $C_2H_5$ | $CH_3$ | 1,2,4-Triazol | Öl |
| 55 | $CH_3$ | $CH_3$ | $C_2H_5$ | Imidazol | Öl |
| 56 | $CH_3$ | $CH_3$ | $C_2H_5$ | 1,2,4-Triazol | 103 |
| 57 | $CH_3$ | $C_2H_5$ | $C_2H_5$ | Imidazol | Öl |
| 58 | $CH_3$ | $C_2H_5$ | $C_2H_5$ | 1,2,4-Triazol | 68 |
| 59 | $C_2H_5$ | $C_2H_5$ | $C_2H_5$ | Imidazol | Öl |
| 60 | $C_2H_5$ | $C_2H_5$ | $C_2H_5$ | 1,2,4-Triazol | 80 |
| 61 | $C_2H_5$ | $C_2H_5$ | $C_2H_5$ | Pyrazol | $Kp_{0,1}$ = 135 |
| 62 | $CH_3$ | $CH_3$ | $C_2H_5$ | Pyrazol | 94-96 |
| 63 | $CH_3$ | $CH_3$ | $-OCH_3$ | Pyrazol | 91-94 |
| 64 | $CH_3$ | $CH_3$ | $-OCH_3$ | Imidazol | |
| 65 | $CH_3$ | $CH_3$ | $-OCH_3$ | 1,2,4-Triazol | 110 |
| 66 | $CH_3$ | $CH_3$ | $-OC_2H_5$ | 1,2,4-Triazol | 57-59 |
| 67 | $C_2H_5$ | $C_2H_5$ | $-OC_2H_5$ | 1,2,4-Triazol | |

| Lfd.Nr. | $R^1$ | $R^2$ | $R^4$ | A | Physikalische Konstante (Fp. °C) |
|---|---|---|---|---|---|
| 68 | $CH_3$ | $CH_3$ | $-OC_3H_7-i$ | 1,2,4-Triazol | |
| 69 | $CH_3$ | $CH_3$ | $-OC_3H_7-i$ | Imidazol | |
| 70 | $CH_3$ | $CH_3$ | -O-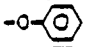 | Pyrazol | 91-92 |
| 71 | $CH_3$ | $CH_3$ | -O-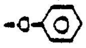 | Imidazol | Harz |
| 72 | $CH_3$ | $CH_3$ | -O-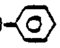 | 1,2,4-Triazol | 112-114 |
| 73 | $C_2H_5$ | $C_2H_5$ | -O-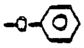 | Pyrazol | |
| 74 | $C_2H_5$ | $C_2H_5$ | -O-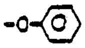 | Imidazol | 115-116 |
| 75 | $C_2H_5$ | $C_2H_5$ | -O-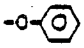 | 1,2,4-Triazol | Öl |
| 76 | $C_2H_5$ | $C_2H_5$ | $-O-CH_2-$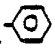 | 1,2,4-Triazol | |
| 77 | $CH_3$ | $CH_3$ | $-CH_2-OCH_3$ | Pyrazol | 113 |
| 78 | $CH_3$ | $CH_3$ | $-CH_2-OCH_3$ | Imidazol | $n_D^{25}$ = 1,582 |
| 79 | $CH_3$ | $CH_3$ | $-CH_2-OCH_3$ | 1,2,4-Triazol | 87 |
| 80 | $C_2H_5$ | $C_2H_5$ | $-CH_2-OCH_3$ | Pyrazol | 74 |
| 81 | $C_2H_5$ | $C_2H_5$ | $-CH_2-OCH_3$ | Imidazol | Öl |
| 82 | $C_2H_5$ | $C_2H_5$ | $-CH_2-OCH_3$ | 1,2,4-Triazol | Öl |
| 83 | $CH_3$ | $CH_3$ | Benzyl | Imidazol | 117-118 |
| 84 | $CH_3$ | $CH_3$ | Benzyl | 1,2,4-Triazol | 40 |
| 85 | $C_2H_5$ | $C_2H_5$ | Benzyl | 1,2,4-Triazol | |
| 86 | $CH_3$ | $CH_3$ | $CH_2-O-C_2H_5$ | 1,2,4-Triazol | |
| 87 | $C_2H_5$ | $C_2H_5$ | $CH_2-OC_3H_7i$ | 1,2,4-Triazol | |
| 88 | $CH_3$ | $CH_3$ | $CH_2-O-CH_2-$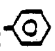 | 1,2,4-Triazol | |
| 89 | $CH_3$ | $CH_3$ | $CH_2-O-$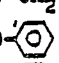 | Pyrazol | |
| 90 | $CH_3$ | $CH_3$ | $CH_2-O-$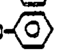 | Imidazol | 84 |
| 91 | $CH_3$ | $CH_3$ | $CH_2-O-$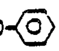 | 1,2,4-Triazol | 110-112 |
| 92 | $C_2H_5$ | $C_2H_5$ | $CH_2-O-$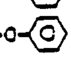 | 1,2,4-Triazol | |
| 93 | $CH_3$ | $CH_3$ | -CH-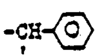 $CH_3$ | 1,2,4-Triazol | |
| 94 | $C_2H_5$ | $C_2H_5$ | -CH-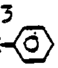 $CH_3$ | 1,2,4-Triazol | 86-88 |
| 95 | $CH_3$ | $CH_3$ | Phenyl | Pyrazol | 121-125 |
| 96 | $CH_3$ | $CH_3$ | Phenyl | Imidazol | 128 |
| 97 | $CH_3$ | $CH_3$ | Phenyl | 1,2,4-Triazol | 135 |
| 98 | $C_2H_5$ | $C_2H_5$ | Phenyl | Pyrazol | 108 |
| 99 | $C_2H_5$ | $C_2H_5$ | Phenyl | Imidazol | 123 |
| 100 | $CH_3$ | $CH_3$ | 2-Methylphenyl | Imidazol | 129-130 |
| 101 | $CH_3$ | $CH_3$ | 2-Methylphenyl | 1,2,4-Triazol | 190-192 |
| 102 | $CH_3$ | $CH_3$ | 2-Methylphenyl | Pyrazol | 182-184 |
| 103 | $CH_3$ | $CH_3$ | 3-Methylphenyl | Imidazol | |
| 104 | $CH_3$ | $CH_3$ | 3-Methylphenyl | 1,2,4-Triazol | |
| 105 | $C_2H_5$ | $C_2H_5$ | 3-Methylphenyl | Imidazol | |
| 106 | $CH_3$ | $CH_3$ | 4-Methylphenyl | Imidazol | 180 |
| 107 | $CH_3$ | $CH_3$ | 4-Methylphenyl | 1,2,4-Triazol | 162-165 |
| 108 | $CH_3$ | $CH_3$ | 2-Chlorphenyl | 1,2,4,-Triazol | |
| 109 | $CH_3$ | $CH_3$ | 3-Chlorphenyl | Imidazol | 151 |
| 110 | $CH_3$ | $CH_3$ | 3-Chlorphenyl | 1,2,4-Triazol | 114-116 |

| Lfd.Nr. | $R^1$ | $R^2$ | $R^4$ | A | Physikalische Konstante (Fp. °C) |
|---|---|---|---|---|---|
| 111 | $CH_3$ | $CH_3$ | 4-Chlorphenyl | Imidazol | 155-156 |
| 112 | $CH_3$ | $CH_3$ | 4-Chlorphenyl | 1,2,4-Triazol | 131-132 |
| 113 | $CH_3$ | $CH_3$ | 2-Fluorphenyl | Imidazol | 147-149 |
| 114 | $CH_3$ | $CH_3$ | 2-Fluorphenyl | 1,2,4-Triazol | 117-120 |
| 115 | $CH_3$ | $CH_3$ | 3-Fluorphenyl | Imidazol | 105-108 |
| 116 | $CH_3$ | $CH_3$ | 3-Fluorphenyl | 1,2,4-Triazol | 122-124 |
| 117 | $CH_3$ | $CH_3$ | 4-Fluorphenyl | Imidazol | 138-139 |
| 118 | $CH_3$ | $CH_3$ | 4-Fluorphenyl | 1,2,4-Triazol | 104-105 |
| 119 | $CH_3$ | $CH_3$ | 3-Bromphenyl | 1,2,4-Triazol | |
| 120 | $CH_3$ | $CH_3$ | 2-Jodphenyl | Pyrazol | 211-212 |
| 121 | $CH_3$ | $CH_3$ | 2-Jodphenyl | Imidazol | 154-155 |
| 122 | $CH_3$ | $CH_3$ | 2-Jodphenyl | 1,2,4-Triazol | 222-223 |
| 123 | $CH_3$ | $CH_3$ | 3-Cyanphenyl | Imidazol | 104-106 |
| 124 | $CH_3$ | $CH_3$ | 3-Cyanphenyl | 1,2,4-Triazol | 147-149 |
| 125 | $C_2H_5$ | $C_2H_5$ | 3-Cyanophenyl | Imidazol | 116-118 |
| 126 | $C_2H_5$ | $C_2H_5$ | 3-Cyanophenyl | 1,2,4-Triazol | 126-128 |
| 127 | $CH_3$ | $CH_3$ | 2-Nitrophenyl | Imidazol | 127-129 |
| 128 | $CH_3$ | $CH_3$ | 2-Nitrophenyl | 1,2,4-Triazol | 175-180 |
| 129 | $C_2H_5$ | $C_2H_5$ | 2-Nitrophenyl | Imidazol | |
| 130 | $C_2H_5$ | $C_2H_5$ | 2-Nitrophenyl | 1,2,4-Triazol | |
| 131 | $CH_3$ | $CH_3$ | 3-Nitrophenyl | Imidazol | 146-148 |
| 132 | $CH_3$ | $CH_3$ | 3-Nitrophenyl | 1,2,4-Triazol | 113-115 |
| 133 | $C_2H_5$ | $C_2H_5$ | 4-Nitrophenyl | Imidazol | 97-100 |
| 134 | $C_2H_5$ | $C_2H_5$ | 4-Nitrophenyl | 1,2,4-Triazol | 117-120 |
| 135 | $C_3H_7$-i | $C_3H_7$-i | Phenyl | Imidazol | 125 |
| 136 | $C_3H_7$-i | $C_3H_7$-i | Phenyl | 1,2,4-Triazol | 130-132 |
| 137 | $C_2H_5$ | $C_2H_5$ | $OCH_3$ | 1,2,4-Triazol | 58 |
| 138 | $C_3H_7$-i | $C_3H_7$-i | 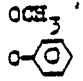 | 1,2,4-Triazol | 163-164 |
| 139 | $C_2H_5$ | $C_2H_5$ | 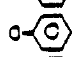 | 1,2,4-Triazol | Öl |
| 140 | $C_2H_5$ | $C_2H_5$ | 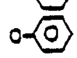 | Imidazol | 115-116 |
| 141 | $CH_3$ | $CH_3$ | 2-Methoxyphenyl | 1,2,4-Triazol | |
| 142 | $CH_3$ | $CH_3$ | 3-Methoxyphenyl | 1,2,4-Triazol | |
| 143 | $CH_3$ | $CH_3$ | 4-Methoxyphenyl | 1,2,4-Triazol | |
| 144 | $C_2H_5$ | $C_2H_5$ | 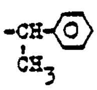 | Imidazol | 75-77 |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von echten Mehltaupilzen und Rostpilzen, z.B. in Getreide, Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora tabacina an Tabak, Plasmopara viticola an Reben, Plasmopara halstedii an Sonnenblumen, Sclerospora macrospora an Mais, Bremia lactucae an Salat, Rhizopus nigricans an Rüben. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzenteile möglich ist.

Als Vergleichsmittel wurde in den folgenden Beispielen der bekannte Wirkstoff

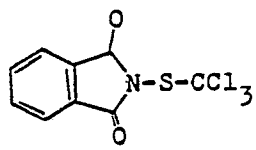

Verbindung A

verwendet.

Die erfindungsgemäßen Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungspektrums wird mit folgenden Fungiziden erzielt:

Manganäthylenbisdithiocarbamat
Mangen-Zinkäthylenbisdithiocarbamat
Ammoniak-Komplex von Zink-(N,N-äthylen-bis-dithiocarbamat)
N-Trichlormethylthio-tetrahydrophthalimid
N-Trichlormethyl-phthalimid
5-Äthoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise:

Dithiocarbamate und deren Derivate, wie
Ferridimethyldithiocarbamat
Zinkdimethyldithiocarbamat
Zinkäthylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
0,0-Diäthyl-phthalimidophonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid

2,3-Dihydro-5-carboxanilido-6-methyl-1,4-oxathiin
2-(Furyl-(2))-benzimidazol
Piperazin-1,4-diylbis-(1)-(2,2,2-trichlor-äthyl)-formamid
2-(Thiazolyl)-(4)-benzimidazol
5-Butyl-2-dimethylamino-4-hydroxy-6-methyl-pyrimidin
Bis-(p-chlorphenyl)-3-pyridinmethanol
1,2-Bis-(3-äthoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyäthyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichloräthan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet. Die Anwendungsformen richten sich ganz nach den Verwendungszwecken ; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht :

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiel 13

Fungizide Wirksamkeit gegen Plasmopara viticola an Reben

Blätter von Topfreben der Sorte Müller-Thurgau werden mit wäßrigen Dispersionen, die 80 % (Gew. %) des zu prüfenden Wirkstoffes und 20 % Natriumligninsulfonat in der Trockensubstanz enthalten, besprüht. Es werden 0,1- und 0,05 %ige Spritzbrühen (bezogen auf die Trockensubstanz) verwendet. Nach dem Antrocknen des Spritzbelages werden die Blätter mit einer Zoosporenaufschwemmung von Plasmopara viticola infiziert. Die Pflanzen kommen dann zuerst für 16 Stunden in eine wasserdampfgesättigte (feuchte) Kammer bei 20 °C und anschließend 8 Tage in ein Gewächshaus mit Temperaturen zwischen 20 und 30 °C. Nach dieser Zeit werden die Pflanzen zur Beschleunigung und Verstärkung des Sporangienträgerausbruches abermals während 16 Stunden in der feuchten Kammer aufgestellt. Dann erfolgt die Beurteilung des Blattbefalls auf den Blattunterseiten. Als Vergleich dienen unbehandelte infizierte Kontrollpflanzen.

O kein Befall, abgestuft bis 5 Totalbefall

| Wirkstoff | Blattbefall nach Spritzung mit ... %iger Spritzbrühe 0,1 | 0,05 |
|---|---|---|
| 64 | 1 | 2 |
| 65 | 1 | 2 |
| 67 | 0 | 1 |
| 69 | 0 | 0 |
| 72 | 1 | 2 |
| 86 | 0 | 0 |
| 89 | 0 | 0 |
| 103 | 0 | 0 |
| 105 | 0 | 0 |
| 135 | 0 | 1 |
| 143 | 1 | 2 |
| A (bekannt) | 2 | 3 |
| Kontrolle (unbehandelt) | 5 | |

Beispiel 14

Fungizide Wirksamkeit gegen Phytophthora infestans an Tomaten

Blätter von Tomatenpflanzen der Sorte « Professor Rudloff » werden mit wäßrigen Suspensionen, die 80 % (Gew. %) des zu prüfenden Wirkstoffes und 20 % Natriumligninsulfonat in der Trockensubstanz enthalten, besprüht. Es wird eine 0,1 %ige Spritzbrühe (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

O kein Befall, abgestuft bis 5 Totalbefall (Kontrolle)

| Wirkstoff | Befall der Blätter nach Spritzung mit 0,1 %iger Spritzbrühe |
|---|---|
| 68 | 2 |
| 69 | 2 |
| 86 | 0 |

| Wirkstoff | Befall der Blätter nach Spritzung mit 0,1 %iger Spritzbrühe |
|---|---|
| 88 | 1 |
| 117 | 0 |
| 133 | 1 |
| Kontrolle (unbehandelt) | 5 |

**Ansprüche** (für die Vertragsstaaten : BE, CH, DE, FR, GB, IT, NL, LU, SE)

1. N-substituiertes Carbonsäureanilid der Formel I

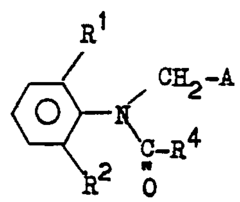

(I)

worin
$R^1$ $C_1$-$C_4$-Alkyl,
$R^2$ $C_1$-$C_4$-Alkyl,
$R^4$ Methoxy, Ethoxy, Isopropoxy, Phenoxy, Benzyl oder 1-Phenyläthyl oder ggf. durch Fluor, Chlor, Brom, Jod, Methyl, Methoxy oder Nitro einfach substituiertes Phenyl bedeutet oder
$R^4$ den Rest $-CH_2-Y$ bedeutet, wobei Y Methoxy, Ethoxy, Phenoxy oder Benzyloxy bedeutet und
A ein über ein Stickstoffatom gebundenes Pyrazol, Imidazol oder 1,2,4-Triazol bedeutet und deren Säureadditionsprodukte.

2. N-1,2,4-Triazolyl-(1)-methyl-methoxyacetyl-2,6-dimethylanilid.

3. Verfahren zur Herstellung eines N-substituierten N-Carbonsäureanilids gemäß Anspruch 1, dadurch gekennzeichnet, daß man 1 Mol eines N-Halogenmethylcarbonsäureanilids der Formel II

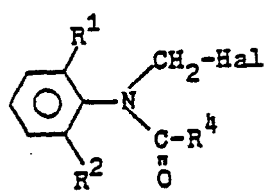

(II)

in welcher $R^1$, $R^2$ und $R^4$ die im Anspruch 1 angegebene Bedeutung haben, und Hal Halogen, insbesondere Chlor oder Brom bedeuten, mit 1 bis 2 Mol eines Azols der Formel III

$$A-M \qquad (III)$$

in welcher A die im Anspruch 1 angegebene Bedeutung hat und M Wasserstoff oder ein Alkalimetall bedeutet, ggf. in Gegenwart eines Verdünnungsmittels und ggf. in Gegenwart von 1,1 bis 1,3 Mol eines Säurebindemittels bei einer Temperatur zwischen −10 und +150 °C umsetzt.

4. Fungizid, enthaltend ein N-substituiertes Carbonsäureanilid gemäß Anspruch 1.

5. Fungizid, enthaltend N-1,2,4-Triazolyl-(1)-methylmethoxyacetyl-2,6-dimethylanilid.

6. Fungizid, enthaltend einen festen oder flüssigen Trägerstoff und ein N-substituiertes Carbonsäureanilid gemäß Anspruch 1.

7. Verfahren zur Herstellung eines Fungizides, dadurch gekennzeichnet, daß man einen festen oder flüssigen Trägerstoff vermischt mit einem N-substituierten Carbonsäureanilid gemäß Anspruch 1.

8. Verfahren zur Bekämpfung von Pilzen, dadurch gekennzeichnet, daß man die Pilze oder die vor Pilzbefall zu schützenden Gegenstände behandelt mit einem N-substituierten Carbonsäureanilid gemäß Anspruch 1.

**Ansprüche** (für den Vertragsstaat AT)

1. Fungizid, enthaltend ein N-substituiertes Carbonsäureanilid der Formel I

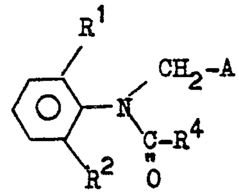

(I)

worin
$R^1$ $C_1$-$C_4$-Alkyl,
$R^2$ $C_1$-$C_4$-Alkyl,
$R^4$ Methoxy, Ethoxy, Isopropoxy, Phenoxy, Benzyl oder 1-Phenyläthyl oder ggf. durch Fluor, Chlor, Brom, Jod, Methyl, Methoxy oder Nitro einfach substituiertes Phenyl bedeutet oder
$R^4$ den Rest $—CH_2—Y$ bedeutet, wobei Y Methoxy, Ethoxy, Phenoxy oder Benzyloxy bedeutet und
A ein über ein Stickstoffatom gebundenes Pyrazol, Imidazol oder 1,2,4-Triazol bedeutet oder deren Säureadditionsprodukte.

2. Fungizid, enthaltend N-1,2,4-Triazolyl-(1)-methylmethoxyacetyl-2,6-dimethylanilid.

3. Fungizid, enthaltend einen festen oder flüssigen Trägerstoff und ein N-substituiertes Carbonsäureanilid wie im Anspruch 1 definiert.

4. Verfahren zur Herstellung eines Fungizides, dadurch gekennzeichnet, daß man einen festen oder flüssigen Trägerstoff vermischt mit einem N-substituierten Carbonsäureanilid wie im Anspruch 1 definiert.

5. Verfahren zur Bekämpfung von Pilzen, dadurch gekennzeichnet, daß man die Pilze oder die vor Pilzbefall zu schützenden Gegenstände behandelt mit einem N-substituierten Carbonsäureanilid wie im Anspruch 1 definiert.

6. Verfahren zur Herstellung eines N-substituierten N-Carbonsäureanilids wie im Anspruch 1 definiert, dadurch gekennzeichnet, daß man 1 Mol eines N-Halogenmethylcarbonsäureanilids der Formel II

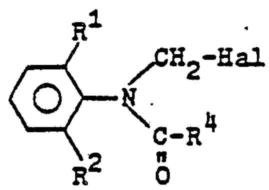

(II)

in welcher $R^1$, $R^2$ und $R^4$ die im Anspruch 1 angegebene Bedeutung haben, und Hal Halogen, insbesondere Chlor oder Brom bedeuten, mit 1 bis 2 Mol eines Azols der Formel III

$$A—M \qquad (III)$$

in welcher A die im Anspruch 1 angegebene Bedeutung hat und M Wasserstoff oder ein Alkalimetall bedeutet, ggf. in Gegenwart eines Verdünnungsmittels und ggf. in Gegenwart von 1,1 bis 1,3 Mol eines Säurebindemittels bei einer Temperatur zwischen −10 und +150 °C umsetzt.

**Claims** (for the contracting States : BE, CH, DE, FR, GB, IT, NL, LU, SE)

1. An N-substituted carboxanilide of the formula I

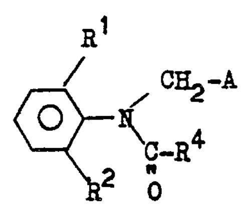

(I)

where

$R^1$ denotes $C_1$-$C_4$-alkyl,

$R^2$ denotes $C_1$-$C_4$-alkyl,

$R^4$ denotes methoxy, ethoxy, isopropoxy, phenoxy, benzyl or 1-phenylethyl or unsubstituted phenyl or phenyl monosubstituted by fluoro, chloro, bromo, iodo, methyl, methoxy or nitro, or

$R^4$ denotes —$CH_2$—Y, Y denoting methoxy, ethoxy, phenoxy or benzyloxy, and

A denotes pyrazole, imidazole or 1,2,4-triazole attached via a nitrogen atom, and its acid addition salts.

2. N-1,2,4-triazolyl-(1)-methyl-methoxyacetyl-2,6-dimethylanilide.

3. A process for the production of an N-substituted N-carboxanilide as claimed in claim 1, characterized in that 1 mole of an N-halomethylcarboxanilide of the formula II

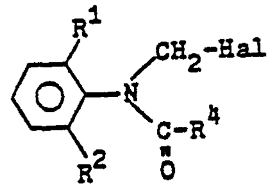

(II)

where $R^1$, $R^2$ and $R^4$ have the meanings given in claim 1, and Hal denotes halogen, especially chlorine or bromine, is reacted with from 1 to 2 moles of an azole of the formula III

$$A—M \qquad (III)$$

where A has the meaning given in claim 1, and M denotes hydrogen or an alkali metal, in the presence or absence of a dilurent and in the presence or absence of 1.1 to 1.3 moles of an acid binder, at from −10° to +150 °C.

4. A fungicide containing an N-substituted carboxanilide as claimed in claim 1.

5. A fungicide containing N-1,2,4-triazol-(1)-methylmethoxyacetyl-2,6-dimethylanilide.

6. A fungicide containing a solid or liquid carrier and an N-substituted carboxanilide as claimed in claim 1.

7. A process for the production of a fungizide, characterized in that a solid or liquid carrier is mixed with an N-substituted carboxanilide as claimed in claim 1.

8. A process for combating fungi, characterized in that the fungi or the objects to be protected against fungus attack are treated with an N-substituted carboxanilide as claimed in claim 1.

**Claims** (for contracting state AT)

1. A fungicide containing an N-substituted carboxanilide of the formula I

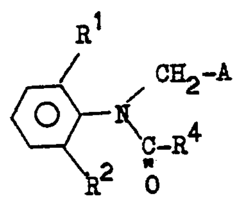

(I)

where

$R^1$ denotes $C_1$-$C_4$-alkyl,

$R^2$ denotes $C_1$-$C_4$-alkyl,

$R^4$ denotes methoxy, ethoxy, isopropoxy, phenoxy, benzyl or 1-phenylethyl or unsubstituted phenyl or phenyl monosubstituted by fluoro, chloro, bromo, iodo, methyl, methoxy or nitro, or

$R^4$ denotes —$CH_2$—Y, Y denoting methoxy, ethoxy, phenoxy or benzyloxy, and

A denotes pyrazole, imidazole or 1,2,4-triazole attached via a nitrogen atom, and its acid addition salts.

2. A fungicide containing N-1,2,4-triazolyl-(1)-methylmethoxyacetyl-2,6-dimethylanilide.

3. A fungicide containing a solid or liquid carrier and an N-substituted carboxanilide as defined in claim 1.

4. A process for the production of a fungicide, characterized in that a solid or liquid carrier is mixed with an N-substituted carboxanilide as defined in claim 1.

5. A process for combating fungi, characterized in that the fungi or the objects to be protected against fungus attack are treated with an N-substituted carboxanilide as defined in claim 1.

6. A process for the production of an N-substituted N-carboxanilide as defined in claim 1, characterized in that 1 mole of an N-halomethylcarboxanilide of the formula II

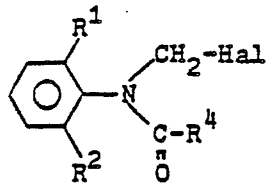

(II)

where $R^1$, $R^2$ and $R^4$ have the meanings given in claim 1, and Hal denotes halogen, especially chlorine or bromine, is reacted with from 1 to 2 moles of an azole of the formula III

$$A—M \qquad (III)$$

where A has the meaning given in claim 1, and M denotes hydrogen or an alkali metal, in the presence or absence of a dilurent and in the presence or absence of 1.1 to 1.3 moles of an acid binder, at from −10° to +150 °C.

**Revendications** (pour les Etats contractants : BE, CH, DE, FR, GB, IT, NL, LU, SE)

1. Anilide d'acide carboxylique N-substitué, de formule I

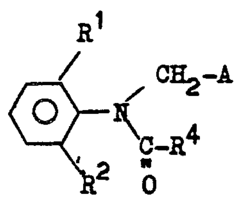

(I)

dans laquelle

$R^1$ représente alkyle en $C_1$-$C_4$

$R^2$ alkyle en $C_1$-$C_4$

$R^4$ méthoxy, éthoxy, isopropoxy, phénoxy, benzyle ou 1-phényléthyle ou phényle substitué une fois par du fluor, chlore, brome, iode, méthyle, méthoxy ou nitro, ou bien

$R^4$ représente le reste —$CH_2$—Y, Y représentant méthoxy, éthoxy, phénoxy ou benzyloxy et

A représente un pyrazole, imidazole ou 1,2,4-triazole lié par un atome d'azote et ses produits d'addition d'acides.

2. N-1,2,4-triazolyl-(1)-méthyl-méthoxyacétyl-2,6-diméthylanilide.

3. Procédé de préparation d'un anilide d'acide carboxylique N-substitué selon la revendication 1, caractérisé par le fait que l'on fait réagir, à une température comprise entre −10 et +150 °C, éventuellement en présence d'un diluant et éventuellement en présence de 1,1 à 1,3 mole d'un liant d'acide, 1 mole d'un anilide d'acide N-halogénométhylcarboxylique de formule II

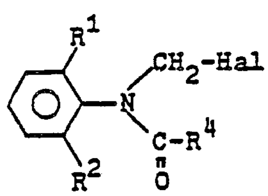

(II)

dans laquelle $R^1$, $R^2$ et $R^4$ ont les significations indiquées dans la revendication 1 et Hal représente un halogène, en particulier chlore ou brome, avec un azole de formule III

$$A—M \qquad (III)$$

dans laquelle A a la signification donnée dans la revendication 1, et M représente l'hydrogène ou un métal alcalin.

4. Fongicide contenant un anilide d'acide carboxylique N-substitué selon la revendication 1.

5. Fongicide contenant un N-1,2,4-triazolyl-(1)-méthylméthoxyacétyl-2,6-diméthylanilide.

6. Fongicide contenant un support solide ou liquide et un anilide d'acide carboxylique N-substitué selon la revéndication 1.

7. Procédé de préparation d'un fongicide, caractérisé par le fait que l'on mélange un support solide ou liquide avec un anilide d'acide carboxylique N-substitué tel que défini à la revendication 1.

8. Procédé de lutte contre les champignons, caractérisé par le fait qu'on traite les champignons, ou les objets à protéger contre les dommages des champignons, avec un anilide d'acide carboxylique N-substitué selon la revendication 1.

**Revendications** (pour l'Etat contractant AT)

1. Fongicide contenant un anilide d'acide carboxylique N-substitué, de formule I

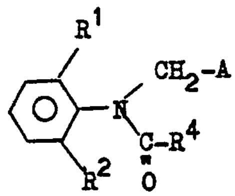

(I)

dans laquelle

$R^1$ représente alkyle en $C_1$-$C_4$

$R^2$ alkyle en $C_1$-$C_4$

$R^4$ méthoxy, éthoxy, isopropoxy, phénoxy, benzyle ou 1-phényléthyle ou phényle substitué une fois par du fluor, chlore, brome, iode, méthyle, méthoxy ou nitro, ou bien

$R^4$ représente le reste $-CH_2-Y$, Y représentant méthoxy, éthoxy, phénoxy ou benzyloxy, et

A représente un pyrazole, imidazole ou 1,2,4-triazole, lié par un atome d'azote et ses produits d'addition d'acides.

2. Fongicide contenant du N-1,2,4-triazolyl-(1)-méthylméthoxyacétyl-2,6-diméthylanilide.

3. Fongicide contenant un support solide ou liquide et un anilide d'acide carboxylique N-substitué selon la revendication 1.

4. Fongicide contenant un anilide d'acide carboxylique N-substitué selon la revendication 1.

5. Procédé de lutte contre les champignons, caractérisé par le fait qu'on traite les champignons, ou les objets à protéger contre les dommages des champignons, avec un anilide d'acide carboxylique N-substitué selon la revendication 1.

6. Procédé de préparation d'un anilide d'acide carboxylique N-substitué selon la revendication 1, caractérisé par le fait que l'on fait réagir, à une température comprise entre −10 et +150 °C, éventuellement en présence d'un diluant et éventuellement en présence de 1,1 à 1,3 mole d'un liant d'acide, 1 mole d'un anilide d'acide N-halogénométhylcarboxylique de formule II

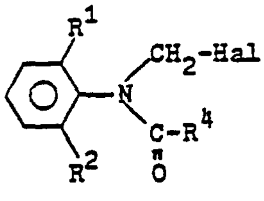

(II)

dans laquelle $R^1$, $R^2$ et $R^4$ ont les significations indiquées dans la revendication 1 et Hal représente un halogène, en particulier chlore ou brome,
avec un azole de formule III

$$A—M \qquad (III)$$

dans laquelle A a la signification donnée dans la revendication 1, et M représente l'hydrogène ou un métal alcalin.